Europäisches Patentamt

European Patent Office

Office européen des brevets

(11) Publication number: **0 442 958 B1**

# EUROPEAN PATENT SPECIFICATION

(45) Date of publication of patent specification: **02.02.94**  (51) Int. Cl.⁵: **A61K 31/22**, A61K 9/08, A61K 47/38

(21) Application number: **89913132.0**

(22) Date of filing: **31.10.89**

(86) International application number:
**PCT/EP89/01304**

(87) International publication number:
**WO 90/04964 (17.05.90 90/11)**

(54) **PHARMACEUTICAL COMPOSITION FOR OPHTHALMIC USE COMPRISING A WATER SOLUBLE ACID ADDITION SALT OF IBOPAMINE.**

(30) Priority: **09.11.88 IT 2255888**

(43) Date of publication of application:
**28.08.91 Bulletin 91/35**

(45) Publication of the grant of the patent:
**02.02.94 Bulletin 94/05**

(84) Designated Contracting States:
**AT BE CH DE FR GB IT LI LU NL SE**

(56) References cited:
**WO-A-86/03970**

(73) Proprietor: **ZAMBON GROUP S.p.A.**
**Via della Chimica, 9**
**I-36100 Vicenza(IT)**

(72) Inventor: **STROPPOLO, Federico**
**Via Vedreggio, 17**
**CH-6963 Pregassona(CH)**
Inventor: **GAZZANIGA, Annibale**
**Via Generale Porro, 22**
**I-20027 Rescaldina(IT)**
Inventor: **CASAGRANDE, Cesare**
**Via Campogallo, 21/67**
**I-20020 Arese(IT)**

(74) Representative: **Marchi, Massimo et al**
**c/o Marchi & Mittler s.r.l.**
**Viale Lombardia 20**
**I-20131 Milano (IT)**

**Description**

The present invention relates to a pharmaceutical composition for ophthalmic use comprising a water soluble pharmaceutically acceptable acid addition salt of ibopamine.

More particularly, the present invention relates to a pharmaceutical aqueous solution for ophthalmic use which is buffered at pH 4.5 and comprises both a water soluble pharmaceutically acceptable acid addition salt of ibopamine and hydroxy propyl methyl cellulose.

It is well known that ibopamine, i.e. epinine 3,4-0-diisobutyrate, is endowed with mydriatic activity (WO 86/03970).

During intensive studies on the properties of aqueous solutions for ophthalmic use comprising a water soluble pharmaceutically acceptable acid addition salt of ibopamine, it has been found that the aqueous solutions of said ibopamine salts such as, for example, hydrochloride, are stable at room temperature for seven days. At lower temperatures the stability of said solutions is slightly greater; in fact, the stability at ± 3°C is of 15 days.

It has now been found that the stability at room temperature is substantially improved when said solutions are buffered at pH 4.5.

Actually, ibopamine titer in aqueous solutions of ibopamine hydrochloride buffered at pH 4.5 remains substantially unchanged for 20-25 days at room temperature and this period of time is sufficient to allow administration of the whole content of a conventional container (i.e. a 5-10 ml small bottle).

### Table I

### 1% solution of ibopamine buffered at different pH

#### Stability data

#### Room Temperature

|          | 1 day | 7 days | 15 days | 20 days |
|----------|-------|--------|---------|---------|
| pH 4.5   | 100%  | 98%    | 95%     | 90%     |
| 6        | 95%   | 79%    | 68%     | ---     |
| 7        | 80%   | 48%    | 35%     | ---     |

### Table II

### 2% solution of ibopamine buffered at pH 4.5

#### Stability data

#### Room Temperature

|        | 1 day | 7 days | 15 days | 20 days |
|--------|-------|--------|---------|---------|
| pH 4.5 | 100%  | 98%    | 95%     | 91%     |

In addition, it has been found that the bioavailability of an aqueous solution of a water soluble salt of ibopamine doubles when said solution contains hydroxy propyl methyl cellulose (also sold under the trademark Methocel - Merck Index X ed., pag. 706, No. 4764).

The evaluation of the mydriatic effect after administration of 1 drop of 1% ibopamine collyrium containing hydroxy propyl methyl cellulose vs. 2% ibopamine collyrium without hydroxy propyl methyl cellulose has been carried out on 13 patients (6 female and 7 male) whose mean age was 50.2 ± 2.7 years; each patient has been treated (single dose) with both collyria at an interval of 7 days between a treatment and the next one. Posology was 1 drop in the right eye; left eye was was not treated (control).

Pupil diameter was measured with a biomicroscope immediately before (zero time) and 30, 60, and 120 minutes after each treatment.

Local tolerability was evaluated on the basis of the following parameters: appearance and degree of burnings and/or of conjunctival hyperemia.

Table III shows the mean results ± e.s.. Maximum pupil dilatation was obtained after 30-60 minutes on the avarage. Meanwhile, the diameter of left eye remained substantially unchanged.

Statistical analysis proves that the two treatments are not significantly different.

```
                              Table III

     Modification of pupil diameter (mm) after treatment with 1%

      ibopamine collyrium containing 0.3% of hydroxy propyl methyl

         cellulose (HPMC) vs. 2% ibopamine collyrium without HPMC.

                       Mean ± e.s. in 13 patients

        Treatment                     Time(minutes)

                           0        30       60       120

        1% ibomamine

           + HPMC        2.39      6.55     7.90     6.79

                        ± 0.04    ± 0.52   ± 0.49   ± 0.39


        2% ibopamine    2.31      6.77     7.93     6.80

                        ± 0.05    ± 0.57   ± 0.52   ± 0.42
```

Therefore, this invention relates to a pharmaceutical aqueous solution for ophthalmic use comprising a water soluble pharmaceutically acceptable acid addition salt of ibopamine, characterized in that said solution is buffered at pH 4.5 and comprises from 0.1 to 0.5 parts by weight of hydroxy propyl methyl cellulose for each part by weight of said ibopamine salt.

The solution of this invention will preferably comprise from 0.5 to 5 parts (w/v) of a water soluble pharmaceutically acceptable acid addition salt of ibopamine; even more preferably they will contain from 1 to 2 parts (w/v) of said ibopamine salt.

Ibopamine hydrochloride is a typical example of a water soluble acid addition salt suitable for preparing the solution of this invention.

The solution of this invention may also comprise from 0.001 to 0.2 parts (w/v) of benzalkonium chloride and from 0.2 to 4 parts (w/v) of mannitol. Furthermore, the solution of this invention may comprise from 0.01 to 0.09 parts (w/v) of EDTA.

Suitable compounds for buffering the solution of this invention are, for example, citric acid and disodium phosphate.

The pharmaceutical composition according to the present invention may comprise other excipients suitable for ophthalmic administration and may be prepared according to conventional methods.

Examples of known containers which may be used in connection with the solution of this invention are those enabling the instant preparation of a sterile solution by a patient in need thereof. A typical package will comprise (i) a small bottle containing a sterile powder or a freeze dried powder, (ii) a vial containing a sterile solvent and (iii) a sterile dropper adapted to fit with said bottle after addition of the solvent to the powder.

A combination of a cap reservoir, dropper and bottle may also be used as described in EP-A-217,425.

This invention relates also to a process for preparing a pharmaceutical composition for ophthalmic use, characterized in that said process comprises distributing a sterile dried water soluble pharmaceutically acceptable acid addition salt of ibopamine in a first sterile container and a substantially aqueous sterile solution having pH 4.5 and comprising from 0.1 to 0.5 parts by weight of hydroxy propyl methyl cellulose for each part by weight of said ibopamine salt in a second sterile container, said sterile solution being adapt to form a mydriatic solution when added to said ibopamine salt before administration to a patient in need of a mydriatic effect.

The following compositions and examples are intended to illustrate the present invention.

| Composition 1 | |
|---|---|
| Ibopamine hydrochloride | 1.000 g |
| Citric acid monohydrate | 0.526 g |
| Dibasic sodium phosphate dodecahydrate | 1.376 g |
| Methocel F4M Premium EP (registered trademark) | 0.300 g |
| Benzalkonium chloride | 0.010 g |
| Mannitol | 2.000 g |
| Sterile water | q.s. to 100 ml |

| Composition 2 | |
|---|---|
| Ibopamine hydrochloride | 1.000 g |
| Citric acid monohydrate | 0.520 g |
| Disodium phosphate dodecahydrate | 1.380 g |
| Methocel F4M Premium EP (registered trademark) | 0.300 g |
| Benzalkonium chloride | 0.010 g |
| Mannitol | 2.000 g |
| EDTA | 0.050 g |
| Sterile water | q.s. to 100 ml |

| Composition 3 | |
| --- | --- |
| Ibopamine hydrochloride | 2.000 g |
| Citric acid monohydrate | 0.351 g |
| Dibasic sodium phosphate dodecahydrate | 0.920 g |
| Methocel F4M Premium EP (registered trademark) | 0.300 g |
| Benzalkonium chloride | 0.010 g |
| Mannitol | 1.333 g |
| Sterile water | q.s. to 100 ml |

## Example 1

### A) Freeze-dried product

composition for

| | | 1 vial | 1,000 vials |
| --- | --- | --- | --- |
| Ibopamine hydrochloride | | mg 60 | g 60 |
| Mannitol | | mg 120 | g 120 |
| Water for injection | q.s. to ml | 1.5 | ℓ 1.5 |

### B) Solvent

| | | 1 vial | 1,000 vials |
| --- | --- | --- | --- |
| Hydroxy propyl methyl cellulose | | mg 18 | g 18 |
| Citric acid monohydrate | | mg 31.6 | g 31.6 |
| Disodium hydrogen phosphate dodecahydrate | | mg 82.8 | g 82.8 |
| Benzalkonium chloride | | mg 0.6 | g 0.6 |
| Water for injection | q.s. to ml | 6 | ℓ 6 |

Ibopamine hydrochloride (60 g) and mannitol (120 g) have been dissolved under stirring in 1,500 ml of water for injection. The solution has been filtered in sterile conditions through a sterile membrane (porosity, 0.2 $\mu$m). In a sterile room, the solution has been distributed in 1,000 sterile vials and these vials have been freeze-dried at the following conditions:
- freezing, plates were cooled at -50°C for 5 hours;
- primary drying, reduced pressure (about 50 $\mu$Bar) for 1 hour; plates were then warmed from -50°C to +20°C in 21 hours (temperature gradient, 0.05°C/min.)
- secondary drying, vials have been maintained at +20°C for five hours, at the end of the treatment the residual pressure was of about 10-15 $\mu$Bar.

Finally, the vials have been plugged in sterile condidions with sterile closures.

The preparation of vials containing the solvent has been carried out as follows: hydroxy propyl methyl cellulose has been dispersed in 2 ℓ of boiling water (for injection). Citric acid monohydrate, disodium hydrogen phosphate dodecahydrate and benzalkonium chloride have been added. The remaining water for injection (4 ℓ) has been cooled and added under stirring and cooling. The clear and viscous solution has been filtered in sterile conditions through a sterile membrane (porosity, 0.2 $\mu$). This solution has been then distributed, in a sterile room, in 1,000 sterile vials which have been closed with sterile closures. Finally the vials have been sterilized in a autoclave at 121°C for 21 minutes.

Example 2

A) Freeze-dried product

composition for

| | | 1 vial | 1,000 vials |
|---|---|---|---|
| Ibopamine hydrochloride | | mg 120 | g 120 |
| Mannitol | | mg 80 | g 80 |
| Water for injection | q.s. to ml | 2 | ℓ 2 |

B) Solvent

| | | 1 vial | 1,000 vials |
|---|---|---|---|
| Hydroxy propyl methyl cellulose | | mg 18 | g 18 |
| Citric acid monohydrate | | mg 21.06 | g 21.06 |
| Disodium hydrogen phosphate dodecahydrate | | mg 55.2 | g 55.2 |
| Benzalkonium chloride | | mg 0.6 | g 0.6 |
| Water for injection | q.s. to ml | 6 | ℓ 6 |

The freeze-dried product (A) and the solvent (B) have been prepared in a way similar to that described in example 1.

**Claims**

1. A pharmaceutical aqueous solution for ophthalmic use comprising a water soluble pharmaceutically acceptable acid addition salt of ibopamine, characterized in that said solution is buffered at pH 4.5 and comprises from 0.1 to 0.5 parts by weight of hydroxy propyl methyl cellulose for each part by weight of said ibopamine salt.

2. A solution according to claim 1, characterized in that Methocel® 4M Premium EP is used as hydroxy propyl methyl cellulose.

3. A solution according to any of the preceding claims 1 and 2, characterized in that said solution comprises from 0.001 to 0.02 parts (w/v) of benzalkonium chloride.

4. A solution according to any of the preceding claims from 1 to 3, characterized in that said solution comprises from 0.2 to 4 parts (w/v) of mannitol.

5. A solution according to any of the preceding claims from 1 to 4, characterized in that said solution comprises from 0.01 to 0.09 parts (w/v) of EDTA.

6. A solution according to any of the preceding claims from 1 to 5, characterized in that ibopamine hydrochloride is the water soluble pharmaceutically acceptable acid addition salt of ibopamine.

7. A solution according to claim 6, characterized in that 100 ml of said solution comprise from 0.5 to 5 g of ibopamine hydrochloride.

**8.** A solution according to claim 7, characterized in that 100 ml of said solution comprise from 1 to 2 g of ibopamine hydrochloride.

**9.** A solution according to claim 8, characterized in that 100 ml of said solution comprise 0.3 g of Methocel F 4M Premium EP.

**10.** A solution according to claim 8, characterized in that 100 ml of said solution comprise 0.05 g of EDTA.

**11.** A solution according to claim 8, characterized in that 100 ml of said solution comprise 0.01 g of benzalkonium chloride.

**12.** A solution according to claim 8, characterized in that 100 ml of said solution comprise 2 g of mannitol.

**13.** A solution according to any of the preceding claims from 1 to 12, characterized in that said solution is instantly preparable by a patient in need thereof.

**14.** A process for preparing a pharmaceutical composition for ophthalmic use, characterized in that said process comprises distributing a sterile dried water soluble pharmaceutically acceptable acid addition salt of ibopamine in a first sterile container and a substantially aqueous sterile solution having pH 4.5 and comprising from 0.1 to 0.5 parts by weight of hydroxy propyl methyl cellulose for each part by weight of said ibopamine salt in a second sterile container, said sterile solution being adapt to form a mydriatic solution when added to said ibopamine salt before administration to a patient in need of a mydriatic effect.

**Patentansprüche**

**1.** Pharmazeutische wäßrige Lösung zur ophthalmologischen Verwendung, umfassend ein wasserlösliches, pharmazeutisch verträgliches Säureadditionssalz von Ibopamin, dadurch gekennzeichnet, daß die Lösung auf einen pH-Wert von 4,5 gepuffert ist und 0,1 bis 0,5 Gewichtsteile Hydroxypropylmethylcellulose je Gewichtsteil Ibopaminsalz umfaßt.

**2.** Lösung nach Anspruch 1, dadurch gekennzeichnet, daß Methocel® 4M Premium EP als Hydroxypropylmethylcellulose verwendet wird.

**3.** Lösung nach einem der vorhergehenden Ansprüche 1 und 2, dadurch gekennzeichnet, daß die Lösung 0,001 bis 0,02 Teile (w/v) Benzalkoniumchlorid umfaßt.

**4.** Lösung nach einem der vorhergehenden Ansprüche 1 bis 3, dadurch gekennzeichnet, daß die Lösung 0,2 bis 4 Teile (w/v) Mannitol umfaßt.

**5.** Lösung nach einem der vorhergehenden Ansprüche 1 bis 4, dadurch gekennzeichnet, daß die Lösung 0,01 bis 0,09 Teile (w/v) EDTA umfaßt.

**6.** Lösung nach einem der vorhergehenden Ansprüche 1 bis 5, dadurch gekennzeichnet, daß Ibopamin-Hydrochlorid das wasserlösliche, pharmazeutisch verträgliche Säureadditionssalz von Ibopamin ist.

**7.** Lösung nach Anspruch 6, dadurch gekennzeichnet, daß 100 ml Lösung 0,5 bis 5 g Ibopamin-Hydrochlorid umfassen.

**8.** Lösung nach Anspruch 7, dadurch gekennzeichnet, daß 100 ml Lösung 1 bis 2 g Ibopamin-Hydrochlorid umfassen.

**9.** Lösung nach Anspruch 8, dadurch gekennzeichnet, daß 100 ml Lösung 0,3 g Methocel F 4M Premium EP umfassen.

**10.** Lösung nach Anspruch 8, dadurch gekennzeichnet, daß 100 ml Lösung 0,05 g EDTA umfassen.

**11.** Lösung nach Anspruch 8, dadurch gekennzeichnet, daß 100 ml Lösung 0,01 g Benzalkoniumchlorid umfassen.

**12.** Lösung nach Anspruch 8, dadurch gekennzeichnet, daß 100 ml Lösung 2 g Mannitol umfassen.

**13.** Lösung nach einem der vorhergehenden Ansprüche 1 bis 12, dadurch gekennzeichnet, daß die Lösung von einem Patienten, der diese benötigt, sofort herstellbar ist.

**14.** Verfahren zur Herstellung eines pharmazeutischen Mittels zur ophthalmologischen Verwendung, dadurch gekennzeichnet, daß man bei diesem Verfahren ein steriles, getrocknetes, wasserlösliches, pharmazeutisch verträgliches Säureadditionssalz von Ibopamin in einem ersten sterilen Behälter und eine im wesentlichen sterile Lösung mit einem pH-Wert von 4,5, die 0,1 bis 0,5 Gewichtsteile Hydroxypropylmethylcellulose je Gewichtsteil Ibopaminsalz umfaßt, in einem zweiten sterilen Behälter verteilt, wobei die sterile Lösung eine pupillenerweiternde Lösung ausbilden kann, wenn sie zu dem Ibopaminsalz hinzugefügt wird, bevor eine Verabreichung an einen Patienten erfolgt, bei dem der pupillenerweiternde Effekt bewirkt werden soll.

**Revendications**

**1.** Solution aqueuse pharmaceutique à usage ophtalmologique comprenant un sel d'addition acide hydrosoluble, pharmaceutiquement acceptable, d'ibopamine, caractérisée en ce que ladite solution est tamponnée à un pH de 4,5 et comprend de 0,1 à 0,5 partie en poids d'hydroxypropylméthylcellulose par partie en poids dudit sel d'ibopamine.

**2.** Solution selon la revendication 1, caractérisée en ce qu'on utilise comme hydroxypropylméthylcellulose du Methocel® 4M Premium EP.

**3.** Solution selon l'une quelconque des revendications précédentes 1 et 2, caractérisée en ce que ladite solution comprend de 0,001 à 0,02 partie (p/v) de chlorure de benzalkonium.

**4.** Solution selon l'une quelconque des revendications précédentes 1 à 3, caractérisée en ce que ladite solution comprend de 0,2 à 4 parties (p/v) de mannitol.

**5.** Solution selon l'une quelconque des revendications précédentes 1 à 4, caractérisée en ce que ladite solution comprend de 0,01 à 0,9 partie d'EDTA.

**6.** Solution selon l'une quelconque des revendications précédentes 1 à 5, caractérisée en ce que le sel d'addition acide hydrosoluble, pharmaceutiquement acceptable, d'ibopamine est le chlorhydrate d'ibopamine.

**7.** Solution selon la revendication 6, caractérisée en ce que 100 ml de ladite solution comprennent de 0,5 à 5 g de chlorhydrate d'ibopamine.

**8.** Solution selon la revendication 7, caractérisée en ce que 100 ml de ladite solution comprennent de 1 à 2 g de chlorhydrate d'ibopamine.

**9.** Solution selon la revendication 8, caractérisée en ce que 100 ml de ladite solution comprennent 0,3 g de Methocel F 4M Premium EP.

**10.** Solution selon la revendication 8, caractérisée en ce que 100 ml de ladite solution comprennent 0,05 d'EDTA.

**11.** Solution selon la revendication 8, caractérisée en ce que 100 ml de ladite solution comprennent 0,01 g de chlorure de benzalkonium.

**12.** Solution selon la revendication 8, caractérisée en ce que 100 ml de ladite solution comprennent 2 g de mannitol.

**13.** Solution selon l'une quelconque des revendications précédentes 1 à 12, caractérisée en ce que ladite solution est préparable instantanément par un malade la nécessitant.

**14.** Procédé de préparation d'une composition pharmaceutique à usage ophtalmologique, caractérisé en ce que ledit procédé comprend la distribution d'un sel d'addition acide hydrosoluble, pharmaceutiquement acceptable, stérile, séché, d'ibopamine dans un premier récipient stérile et d'une solution stérile sensiblement aqueuse ayant un pH de 4,5 et comprenant de 0,1 à 0,5 partie en poids d'hydroxypropylméthylcellulose par partie en poids dudit sel d'ibopamine dans un second récipient stérile, ladite solution stérile étant conçue pour former une solution mydriatique lorsqu'elle est ajoutée audit sel d'ibopamine avant son administration à un malade nécessitant un effet mydriatique.